(19) Europäisches Patentamt
European Patent Office
Office européen des brevets

(11) Publication number: 0 248 466 B1

(12) **EUROPEAN PATENT SPECIFICATION**

(45) Date of publication of the patent specification: 31.10.90

(51) Int. Cl.[5]: **C07D 257/08**, A01N 43/713 // C07C57/56, C07C57/76, C07C249/02, C07C257/00

(21) Application number: 87200917.0

(22) Date of filing: 15.05.87

(54) **Pesticidal tetrazine derivatives.**

(30) Priority: 05.06.86 US 871199
05.06.86 US 871198

(43) Date of publication of application: 09.12.87 Bulletin 87/50

(45) Publication of the grant of the patent: 31.10.90 Bulletin 90/44

(84) Designated Contracting States: DE ES FR GB IT NL

(56) References cited:
EP-A- 0 005 912
EP-A- 0 029 657

(73) Proprietor: SHELL INTERNATIONALE RESEARCH MAATSCHAPPIJ B.V., Carel van Bylandtlaan 30, NL-2596 HR Den Haag(NL)

(72) Inventor: Pilgram, Kurt Hans Gerhard, 2607 Warwick Lane, Modesto California 95350(US)

(74) Representative: Bennett, David Arthur Horder et al, 4, York Road, London SE1 7NA(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to tetrazine derivatives, more particularly 3,6-disubstituted-1,2,4,5-tetrazine compounds, their preparation, and their use as pesticides, particularly as mite ovicides.

EP-A-5912 (Fisons) discloses the acaricidal, particularly larvicidal and ovicidal, activity of tetrazines of the formula

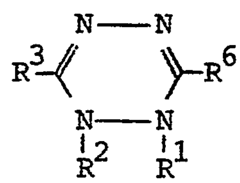

and

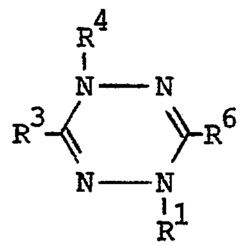

wherein $R^1$, $R^2$ and $R^4$, which may be the same or different, each represent hydrogen, phenyl, alkyl of 1 to 6 carbon atoms or alkenyl or alkynyl of 3 to 6 carbon atoms, each of which phenyl, alkyl, alkenyl or alkynyl groups may be unsubstituted or substituted by one or more halogen atoms, hydroxy groups, cyano groups, carboxy groups, alkoxycarbonyl groups of 2 to 5 carbon atoms or alkoxy groups of 1 to 4 carbon atoms; or $R^1$ and $R^2$ together represents a single bond, $R^3$ represents alkyl of 1 to 6 carbon atoms, cycloalkyl of 3 to 7 carbon atoms, phenyl, or phenylalkyl of 7 to 10 carbon atoms, each of which may be unsubstituted or substituted by one or more halogen atoms, alkyl or alkoxy groups of 1 to 6 carbon atoms, nitro groups, cyano groups, mercapto groups or alkylmercapto groups of 1 to 4 carbon atoms; and $R^6$ represents a phenyl group substituted in at least the 2-position by fluorine, chlorine, bromine or iodine.

EP-A-29657 (Fisons) discloses a class of tetrazine derivatives having pesticidal, particularly acaricidal, larvicidal and ovicidal, activity, of the formulae

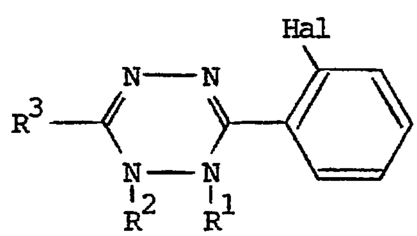

and

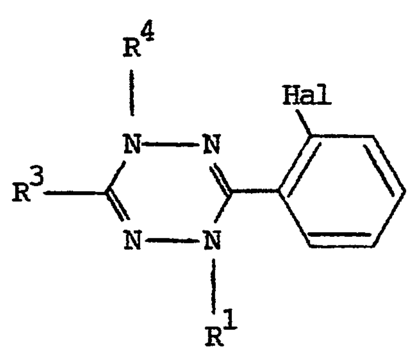

wherein $R^1$, $R^2$ and $R^4$, which may be the same or different, each represent hydrogen, phenyl, alkyl of 1 to 6 carbon atoms, or alkenyl or alkynyl of 3 to 6 carbon atoms, each of which may be unsubstituted or

substituted by one or more halogen atoms, hydroxy groups, cyano groups, carboxy groups, alkoxycarbonyl groups of 2 to 5 carbon atoms or alkoxy groups of 1 to 4 carbon atoms; or $R^1$ and $R^2$ together represent a single bond; Hal represents fluorine, chlorine, bromine or iodine; and $R^3$ represents 2-iodophenyl, 2,5-dihalophenyl, 2-methylthiophenyl, cyclohexyl, cyclohexylalkyl, cyclohexenyl, cyclohexadienyl, 1-naphthyl, 2-naphthyl or biphenylyl, each of the cyclohexyl, cyclohexenyl, cyclohexadienyl, naphthyl or biphenylyl moieties of which may be unsubstituted or substituted by one or more alkyl, alkoxy or alkylthio groups of 1 to 6 carbon atoms, hydroxy, mercapto or cyano groups, halogen atoms, alkanoyloxy groups of 2 to 6 carbon atoms, amino or phenylamino groups, or alkylamino or dialkylamino groups, each alkyl moiety of which is of 1 to 6 carbon atoms.

There has now been discovered a novel class of tetrazine derivatives exhibiting useful pesticical, particularly mite ovicidal activity.

According to the present invention there is provided a 3, 6-disubstituted-1,2,4,5-tetrazine compound of formula

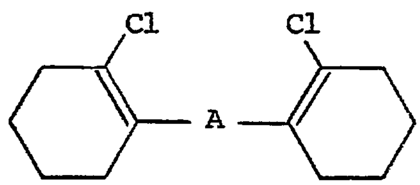

(I)

wherein A is a 1,2,4,5-tetrazinylene group or is a 1,4-dihydro-1,2,4,5-tetrazinylene group of formula

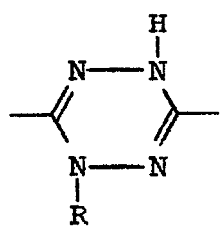

(II)

wherein R represents a hydrogen atom, a $C_{1-4}$ alkyl group, a ($C_{1-6}$ alkoxy)methyl group, a ($C_{2-6}$ alkoxyalkoxy)methyl group, a phenyl $C_{1-4}$ alkyl group or a halophenyl $C_{1-4}$ alkyl group.

When A is a group of formula II, R preferably represents a hydrogen atom, a straight-chain $C_{1-4}$ alkyl group, a benzyl group or a 2-halobenzyl group.

The term "halo" herein embraces fluorine, chlorine and bromine, fluorine being preferred.

Most preferred compounds of formula I are 3,6-bis (2-dichloro-1-cyclohexen-1-yl)-1,2,4,5-tetrazine and the compounds wherein A is a group of formula II and R is a hydrogen atom, or a methyl, ethyl, benzyl or 2-fluorobenzyl group.

The invention further provides a process for preparing a compound of formula I as defined above which comprises reacting a compound of formula.

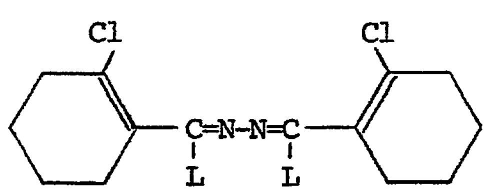

(III)

wherein L is a leaving group, preferably a chlorine atom, with a hydrazine of formula $RNH-NH_2$ wherein R is as defined above, in the presence of an inert solvent, and isolating the desired compound of formula I, optionally followed when R is hydrogen by reduction of resulting compound of formula I wherein A is the group of formula II and R is hydrogen to the compound of formula I wherein A is a 1,2,4,5-tetrazinylene group.

Compounds of formula III may be prepared as described specifically hereinafter wherein each L moiety is a chlorine atom, or by analogous methods, starting from 2-chloro-1-formyl-1-cyclohexene, the preparation of which is described in Synthesis 1981 (6) pp 483-4.

Hydrazines of formula $RNH-NH_2$ wherein R is hydrogen, $C_{1-4}$alkyl or benzyl are known materials. Those wherein R is halobenzyl may be prepared according to the method described specifically hereinafter for (2-fluorobenzyl) hydrazine. Hydrazines wherein R is ($C_{1-6}$ alkoxy)methyl or ($C_{2-6}$ alkoxy-

alkoxy)methyl can be prepared by treating the corresponding chloromethyl (C $_{1-6}$ alkyl) ether or chloromethyl (C $_{2-6}$ alkoxyalkyl) ether with a two-to-four- molar excess of hydrazine. Alternatively, an alkyl hydrazine carboxylate ("alkyl carbazate") can be alkylated with the chloromethyl (C $_{1-6}$ alkyl or C $_{2-6}$ alkoxyalkyl) ether and the resulting 2-(C $_{1-6}$ alkoxy)methyl (or (C $_{2-6}$ alkoxyalkoxy)methyl) hydrazinecarboxylate can be treated with a base to hydrolyze/decarboxylate it to give the (C $_{1-6}$ alkoxy)methyl- or (C $_{2-6}$ alkoxyalkoxy)methyl hydrazine.

Reaction of the compound of formula III with the hydrazine of formula $RNH-NH_2$ may conveniently be effected at a temperature in the range from ambient temperature to reflux temperature of the reaction mixture. Suitable inert solvents include alkanols, e.g. ethanol. Reduction of a compound of formula I wherein A is the group of formula II wherein R is hydrogen to the compound of formula I which A is a 1,2,4,5-tetrazinylene group may conveniently be effected in aqueous solution using an alkali metal nitrite, e.g. sodium nitrite, in the presence of an acid such as acetic acid, e.g. at a temperature in the range 0 to 15°C.

Compounds of formula I have been found to be toxic to the eggs (ova) of mites and consequently are of interest for combating mites that attack plants. The invention thus also provides a method for combating pests at a locus e.g. mites that attack plants, that method comprising applying an effective amount of a compound of formula I to that locus, e.g. to a plant either infested with mite eggs or liable to infestation with them.

For application, the compound of formula I ordinarily is applied most effectively by formulating it with a suitable inert carrier or surface-active agent, or both. The invention, therefore, also includes compositions suitable for combating mites, such compositions comprising an inert carrier or surface-active agent, or both, and a compound of formula I as active ingredient.

The term "carrier" as used herein means an inert solid or liquid material, which may be inorganic or organic and of synthetic or natural origin with which the active compound is mixed or formulated to facilitate its application to the plant to be treated, or its storage, transport and/or handling. Any of the materials customarily employed in formulating pesticides, i.e. horticulturally acceptable adjuvants, are suitable.

Suitable solid carriers are natural and synthetic clays and silicates, for example, natural silicas such as diatomaceous earths; magnesium silicates, for example talcs; magnesium aluminium silicates, for example, attapulgites and vermiculites; aluminium silicates, for example, kaolinites, montmorillonites a nd micas; calcium carbonate; calcium sulfate; synthetic hydrated silicon oxides and synthetic calcium or aluminium silicates; elements such as, for example, carbon and sulfur; natural and synthetic resins such as, for example, coumarone resins, polyvinyl chloride and styrene polymers and copolymers; bitumen; waxes such as, for example, beexwax, paraffin wax, and chlorinated mineral waxes; and ground, naturally-occurring, fibrous materials, such as ground corncobs.

Examples of suitable liquid carriers are water, alcohols such as isopropyl alcohol and glycols; ketones such as acetone, methyl ethyl ketone, methyl isobutyl ketone and cyclohexanone; ethers such as 2-ethoxyethanol; aromatic hydrocarbons such as benzene, toluene and xylene; petroleum fractions such as kerosene, light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, solutions, emulsifiable concentrates, emulsions, suspension concentrates and aerosols. Wettable powders are usually compounded to contain 25-75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Aqueous dispersions and emulsions, for example, com positions obtained by diluting a wettable powder or a concentrate according to the invention with water, also lie within the scope of the present invention. The said emulsions may be of the water-in-oil or the oil-in-water type, and may have thick, meyonnaise-like consistency.

It is evident from the foregoing that this invention contemplates compositions containing as little as about 0.0001% by weight to as much as about 95% by weight of a compound of the invention as the active ingredient.

The compositions of the invention may also contain other ingredients, for example, other compounds possessing pesticidal, especially insecticidal, acaricidal or fungicidal properties, as are appropriate to the intended purpose.

The method of applying a compound of formula I to combat mites comprises applying the compound, ordinarily in composition of one of the aforementioned types, to a locus or area to be protected from the mites, such as the foliage and/or the fruit of plants. The compound can be applied to plants already infested with ova of the mites, or the compound can be applied beforehand to plants that are expected to become infested with mites. The compound, of course, is applied in an amount sufficient to effect the desired action. This dosage is dependent upon any factors, including the carrier employed, the method and conditions of the application, whether the formulation is present at the locus in the form of an aerosol, or as a film, or as discrete particles, the thickness of film or size of particles. Proper consideration and resolution of these factors to provide the necessary dosage of a compound of formula I at the locus to be protected are within the skill of those versed in the art. In general, however, the effective dosage of a compound of formula I at the locus to be protected - i.e., the dosage which the mite contacts - is of the order of 0.001 to 0.5% based on the total weight of the formulation, though under some circumstances the effective concentration will be as little as 0.0001% or as much as 2%, on the same basis.

The invention will be further understood from the following examples, in which identities of various products and precursors were confirmed as necessary by appropriate chemical and spectral analyses.

## EXAMPLE 1

Preparation of 3,6-bis(2-chloro-1-cyclohexen-1-yl)-1,4-dihydro-1,2,4,5-tetrazine (Compound 1) and 3,6-bis (2-chloro-1-cyclohexen-1-yl)-1,2,4,5-tetrazine (Compound 2)

A solution of 49 g of silver nitrate in 270 ml of water was stirred vigorously at ambient temperature(20°C) while a strong stream of gaseous oxygen was bubbled into the mixture. Then 40 ml of a solution of 50 g of sodium hydroxide in 280 ml of water, and 12 ml of 30% hydrogen peroxide in water were both added drop-by-drop. The temperature of the mixture was brought to 25°C and 102.5 g of 2-chloro-1-formyl-1-cyclohexene and 240 ml of the sodium hydroxide solution were added drop-by-drop to the stirred mixture, the mixture being cooled to hold its temperature at 29°C. When the reaction was complete (i.e. when the aldehyde had been consumed, as determined by gas-liquid chromotography), the mixture was filtered, and the aqueous filtrate was extracted with ether. The remaining aqueous phase was acidified with sulfuric acid and filtered. The collected crystals were washed with ice-water, dried in a vacuum oven at 25°C, and recrystallized from hexane, to give 2-chloro-l-cyclohexene-1-carboxylic acid ( 1A), as yellow crystals, m.p. 107-107.5°C.

At ambient temperature (20°C), 5 drops of dimethylformamide were added to a stirred mixture of 43 g of 1A and 500 ml of thionyl chloride, and the mixture was heated at reflux (77°C) for 6 hours. Unreacted thionyl chloride was stripped from the mixture, to give 2-chloro-1 cyclohexene-1-carboxylic acid chloride ( 1B), as a brown liquid.

A solution of 14.4 g o f 95% hydrazine in 25 ml of tetrahydrofuran (THF) was added drop-by-drop over 30 minutes to a vigorously stirred mixture of 50g of 1B and 500 ml of THF, the temperature of the mixture being held at 0-5°C. Then the mixture was stirred for 30 minutes at 0°C, allowed to warm to ambient temperature and poured into 2500 ml of stirred ice water. The resulting mixture was filtered and the collected solid was dried in a vacuum oven to give 1,2-bis(2-chloro-1-cyclohexene-1-carbonyl) hydrazine ( 1C), as a tan solid, m.p. 255-258°C.

35g of 1C was added over 5 minutes to a stirred solution of 45.8 g of phosphorus pentachloride in 800 ml of phosphorus oxychloride, at ambient temperature. The resulting mixture was stirred at 70°C for one hour, then at reflux (107°C) for 2 hours (until evolution of hydrogen chloride ceased), then the volatiles were stripped under reduced pressure and the residue was poured into 2500 ml of a vigorously stirred ice/water mixture. The resulting mixture was extracted with methylene chloride. The extract was dried ($MgSO_4$), filtered and stripped of solvent to give 1,4-dichloro-1,4-bis(2-chloro-1-cyclohexen-1-yl) azine ( 1D), as a heterogeneous brown syrup.

At 0-5°C, 11.9 g of 95% hydrazine was added drop-by-drop over 15 minutes to a stirred solution of 17.5 g of 1D in 200 ml of absolute ethanol. The mixture was heated at reflux for 10 minutes, then stirred at ambient temperature for 18 hours. The mixture was filtered, the filtrate was stripped of solvent, and the residue was poured into 2500 ml of a vigorously stirred ice/water mixture. The resulting mixture was extracted with ether, the extract was dried ($MgSO_4$) and stripped of solvent, and the residue was absorbed on silica gel for dry column chromatography. The chromatography was performed with a 1:4:20 v:v:v mixture of THF, ethyl acetate and hexane as eluent. Three crystalline fractions were obtained of which Compound 1 was a yellow solid, m.p.: 198 to 202°C, when recrystallized from hexane, and Compound 2 was a red-purple solid, m.p.: 165 to 169°C, when recrystallised from hexane.

EXAMPLE 2

Preparation of Compound 1

1D was reprepared as described in Example 1, from a purified sample of 1C.

A solution of 7 g of 1D in 10 ml of THF was added drop-by-drop to a stirred mixture of 3.4 g of 97% (essentially anhydrous) hydrazine and 100 ml of absolute ethanol at ambient temperature, then the mixture was stirred at reflux for 30 minutes. Then the solvent was stripped under reduced pressure and the residue was dissolved in 150 ml of boiling toluene. The solution was allowed to cool to 25°C and filtered, giving Compound 1 as a colorless solid, m.p.: 202-205°C. Air was excluded throughout the procedures.

EXAMPLE 3

Preparation of Compound 2

Excess of a 20% aqueous solution of sodium nitrite was added drop-by-drop to a stirred solution of 1.5 g of Compound 1 from Example 2 and 25 ml of glacial acetic acid at 10-15°C. Ice water was added and the resulting mixture was filtered. The collected solid was washed with cold water, air-dried and recrystallized from a 1:1 v:v mixture of diethyl ether and hexane to give Compound 2 as a red solid, m.p.: 166-169°C.

EXAMPLES 4 to 6

By the procedures described in Examples 1 and 2, but substituting, in place of the hydrazine, methylhydrazine, ethylhydrazine, and benzylhydrazine respectively, there were prepared:

3,6-bis(2-chloro-1-cyclohexen-1-yl)-1-methyl-1,4-dihydro- ,2,4,5-tetrazine (Compound 3), yellow crystals, m.p.:129 to 131°C,

3,6-bis(2-chloro-1-cyclohexen-1-yl)-1-ethyl-1,4-dihydro-1,2, ,5-tetrazine (Compound 4), yellow solid, m.p.: 97 to 99°C, and 3,6-bis(2-chloro-1-cyclohexen-1-yl)-1-benzyl-1,4-dihydro-1,2,4,5-tetrazine (Compound 5), yellow solid, m.p.: 95-97°C.

EXAMPLE 7

Preparation of 3,5-bis(2-chloro-1-cyclohexen-1-yl)-1-(2-fluorobenzyl)-1,4-dihydro-1,2,4,5-tetrazine (Compound 6)

27 g of methyl hydrazinocarboxylate was added to a mixture of 37.2 g of 2-fluorobenzaldehyde, 125 ml of methanol and 150 ml of water. The mixture was warmed to 64°C, then heated at reflux for 15 minutes, cooled to 0°C and filtered; the solid material was methyl 3-((2-fluoropheny1) methylene)carbazate ( 6A), m.p.: 135-137°C.

A mixture of 53 g of 6A and 250 ml of methanol was cooled to 0°C. A small amount of erythrosin was added and the mixture was adjusted to pH = 3-4 by addition of 2N hydrochloric acid. Then 11.4 g of sodium cyanoborohydride was added in portions over 30 minutes to the stirred mixture, hydrochloric acid being added dropwise to keep the pH at 3-4. The mixture was stirred at ambient temperature for 3 hours, then stripped of solvent under reduced pressure. The residue was mixed with 250 ml of cold water (0°C) and the resulting mixture was extracted with ether. The extract was dried ($MgSO_4$), filtered and stripped of solvent, to give methyl 3-(2-fluorobenzyl) carbazate ( 6C), as a colorless syrup.

A solution of 46 g of 6C in 50 ml of methanol was added to a mixture of 20 g of sodium hydroxide in 250 ml of water. The resulting mixture was stirred and heated at reflux for 2 hours, cooled to 10°C and, after treatment with concentrated hydrochloric acid unit the mixture was distinctly acidic, was extracted with ether. The aqueous fraction was made distinctly basic by addition of 50% aqueous sodium hydroxide solution and extracted with ether. The extract was dried ($MgSO_4$) and filtered. The filtrate was stripped of solvent to give (2-fluorobenzyl) hydrazine ( 6D), as a light tan clear syrup.

Compound 6 was prepared, as an amber syrup, from 6D according to the procedures described in Example 2 for preparing Compound 1 from 1D.

EXAMPLE 8

ACTIVITY TESTS

Activity of individual species of formula I with respect to ova of mites was determined using standardized test methods to measure the toxicity of the compound as follows:

1. Toxicity to ova - acute

Young pinto bean plants, Phaseolus vulgaris, were trimmed to a single leaf and infested with adult female mites, Tetranychus urticae. After 18 hours, all motile forms were removed from the leaf with a gentle stream of air, leaving only the eggs. Both the upper and lower surfaces of the leaf were treated with 2 milliters of the test solution prepared by diluting a 1% w/v acetone stock solution of the test compound with 0.055% "Atlox 1045A" (trade mark) surfactant in water to the desired concentration. The test solution was sprayed on the foliage of the plant as it was rotating on a turntable. Treated plants were held for 5 days at 80°F (27°C) and 55% relative humidity, at which time the leaves were inspected for live mites and unhatched eggs. the percent egg mortality was calculated as follows:

$$\% \text{ Egg Mortality} = \frac{\text{Number of Unhatched Eggs}}{\text{Number of Live Mites} + \text{Number of Unhatched Eggs}} \times 100$$

The compound was tested at different dosages, and the $LC_{50}$ dosage (percent of the compound in the test solution) was determined with a probit analysis program.

The results were as follows:

Table 1

| Compound No | $LC_{50}$ dosage (%) |
|---|---|
| 1 | 0.001 |
| 2 | 0.002 |
| 3 | 0.006 |
| 4 | 0.006 |
| 5 | 0.002 |
| 6 | 0.001 |

II. Toxicity of Ova - Residual

Two sets of uninfested plants were sprayed with solutions of the test compounds as described in I, above, and held as described above. In one set, one day later the plants were infested with female mites, 18-24 hours later the motile forms were removed, and the eggs were allowed to develop for 4-5 days. Then the numbers of unhatched eggs were counted and the $LC_{50}$ values were calculated.

The other set was treated in the same way, except that the plants were infested seven days after they had been sprayed.

The results were as follows:

Table II

| Compound No. | $LC_{50}$dosage (%) | |
|---|---|---|
| | 1 day | 7 days |
| 1 | 0.001 | 0.046 |
| 2 | 0.0055 | 0.069 |
| 3 | 0.042 | 0.237 |
| 4 | 0.027 | 0.129 |
| 5 | 0.024 | 0.44 |
| 6 | 0.021 | 0.106 |

**Claims for the Contracting States: DE, FR, GB, IT, NL**

1. A 3,6-disubstituted-1,2,4,5-tetrazine compound of formula

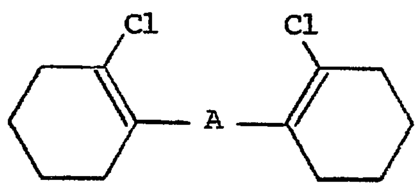

(I)

wherein A is a 1,2,4,5-tetrazinylene group or is a 1,4-dihydro-1,2,4,5-tetrazinylene group of formula

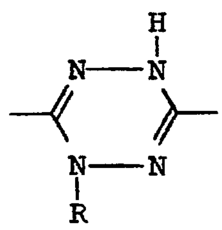

(II)

wherein R represents a hydrogen atom, a C $_{1-4}$ alkyl group, a (C $_{1-6}$ alkoxy)methyl group, a (C $_{2-6}$ alkoxy-alkoxy) methyl group, a phenyl C $_{1-4}$ alkyl group or a halophenyl C $_{1-4}$ alkyl group.

2. A compound according to Claim 1 wherein when A is a group of formula II R represents a hydrogen atom, a straight-chain C $_{1-4}$ alkyl group, a benzyl group or a 2-halobenzyl group.

3. A compound according to Claim 1 or 2 wherein when A is a group of formula II R is a hydrogen atom, or a methyl, ethyl, benzyl or 2-fluorobenzyl group.

4. A process for preparing a compound of formula I as defined in any one of Claims 1 to 3 which comprises reacting a compound of formula.

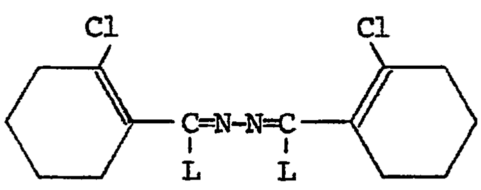

(III)

wherein L is a leaving group, with a hydrazine of formula $RNH-NH_2$ wherein R is as defined in Claim 1, in

the presence of an inert solvent, and isolating the desired compound of formula I, optionally followed when R is hydrogen by reduction of resulting compound of formula I wherein A is the group of formula II and R is hydrogen to the compound of formula I wherein A is a 1,2,4,5-tetrazinylene group.

5. A process according to Claim 4 wherein L is a chlorine atom.

6. A pesticidal composition comprising at least one carrier and, as active ingredient, a compound of formula I as claimed in any one of Claims 1 to 3.

7. A composition accoording to Claim 6 which comprises at least two carriers, at least one of which is a surface active agent.

8. A method of combating pests at a locus which comprises applying to the locus a pesticidally effective amount of a compound as claimed in any one of Claims 1 to 3 or a composition as claimed in Claim 6 or 7.

**Claims for the Contracting State: ES**

1. A process for preparing a 3,6-disubstituted-1,2,4,5-tetrazine compound of formula

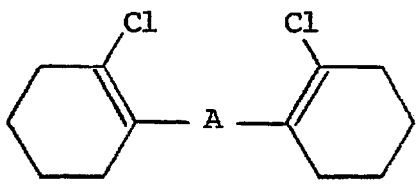

(I)

wherein A is a 1,2,4,5-tetrazinylene group or is a 1,4-dihydro-1,2,4,5-tetrazinylene group of formula

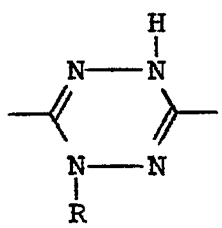

(II)

wherein R represents a hydrogen atom, a $C_{1-4}$ alkyl group, a ($C_{1-6}$ alkoxy)methyl group, a ($C_{2-6}$ alkoxyalkoxy) methyl group, a phenyl $C_{1-4}$ alkyl group or a halophenyl $C_{1-4}$ alkyl group, which comprises reacting a compound of formula

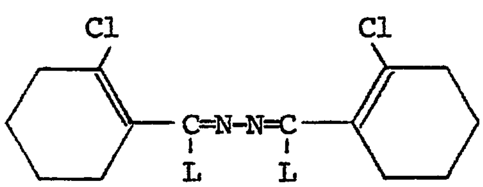

(III)

wherein L is a leaving group, with a hydrazine of formula $RNH–NH_2$ wherein R is as previously defined, in the presence of an inert solvent, and isolating the desired compound of formula I, optionally followed when R is hydrogen by reduction of resulting compound of formula I wherein A is the group of formula II and R is hydrogen to the compound of formula I wherein A is a 1,2,4,5-tetrazinylene group.

2. A process according to Claim 1 wherein when A is a group of formula II R represents a hydrogen atom, a straight- chain $C_{1-4}$ alkyl group, a benzyl group or a 2-halobenzyl group.

3. A process according to Claim 1 or 2 wherein when A is a group of formula II R is a hydrogen atom, or a methyl, ethyl, benzyl or 2-fluorobenzyl group.

4. A process according to Claim 1, 2 or 3 wherein L is a chlorine atom.

5. A method of combating pests at a locus which comprises applying to the locus a pesticidally effective amount of a compound of formula I as defined in any one of Claims 1 to 3.

**Patentansprüche für die Vertragsstaaten: DE, FR, GB, IT, NL**

1. 3,6-Disubstituierte-1,2,4,5-tetrazinverbindung der Formel

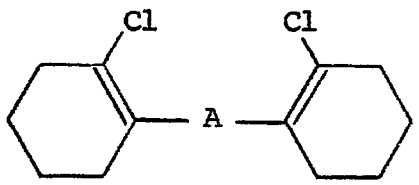

(I)

worin A eine 1,2,4,5-Tetrazinylengruppe oder eine 1,4-Dihydro-1,2,4,5-tetrazinylengruppe der Formel

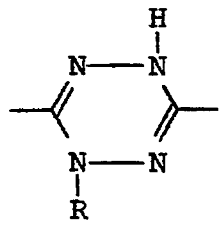

(II)

ist, worin R für ein Wasserstoffatom, eine $C_{1-4}$-Alkylgruppe, eine ($C_{1-6}$-Alkoxy)methylgruppe, eine ($C_{2-6}$-Alkoxyalkoxy)methylgruppe, eine Phenyl-$C_{1-4}$-alkylgruppe oder eine Halophenyl-$C_{1-4}$-alkylgruppe steht.

2. Verbindung nach Anspruch 1, worin R ein Wasserstoffatom, eine geradkettige $C_{1-4}$-Alkylgruppe, eine Benzylgruppe oder eine 2-Halobenzylgruppe darstellt, wenn A eine Gruppe der Formel II ist.

3. Verbindung nach Anspruch 1 oder 2, worin R ein Wasserstoffatom oder eine Methyl-, Ethyl-, Benzyl- oder 2-Fluorbenzylgruppe ist, wenn A eine Gruppe der Formel II ist.

4. Verfahren zur Herstellung einer Verbindung der Formel I nach einem der Ansprüche 1 bis 3, umfassend das zur Reaktionbringen einer Verbindung der Formel

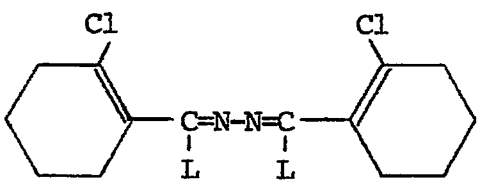

(III)

worin L eine Abgangsgruppe ist, mit einem Hydrazin der Formel $RNH{-}NH_2$, worin R wie in Anspruch 1 definiert ist, in Gegenwart eines inerten Lösungsmittels und die Isolierung der gewünschten Verbindung der Formel I, gegebenenfalls, wenn R Wasserstoff ist, gefolgt von einer Reduktion der erhaltenen Verbindung der Formel I, worin A die Gruppe aus Formel II ist und R Wasserstoff ist, zu der Verbindung von Formel I, worin A eine 1,2,4,5-Tetrazinylengruppe ist.

5. Verfahren nach Anspruch 4, worin L ein Chloratom ist.

6. Schädlingsbekämpfungsmittelzusammensetzung, umfassend mindestens einen Träger und als aktiven Bestandteil eine Verbindung der Formel I nach einem der Ansprüche 1 bis 3.

7. Zusammensetzung nach Anspruch 6, umfassend mindestens zwei Träger, wobei mindestens einer von diesen ein oberflächenaktives Mittel ist.

8. Verfahren zur Bekämpfung von Schädlingen an einem Ort, umfassend die Applikation einer für die Schädlingsbekämpfung wirksamen Menge einer Verbindung nach einem der Ansprüche 1 bis 3 oder einer Zusammensetzung nach Anspruch 6 oder 7 an diesen Ort.

**Patentansprüche für den Vertragsstaat: ES**

1. Verfahren zur Herstellung einer 3,6-disubstituierten1,2,4,5-Tetrazinverbindung der Formel

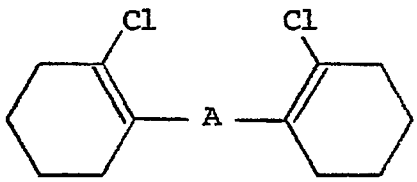

(I)

worin A eine 1,2,4,5-Tetrazinylengruppe oder eine 1,4-Dihydro-1,2,4,5-tetrazinylengruppe der folgenden Formel ist

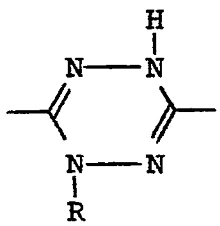

(II)

worin R für ein Wasserstoffatom, eine $C_{1-4}$-Alkylgruppe, eine ($C_{1-6}$-Alkoxy)methylgruppe, eine ($C_{2-6}$-Alkoxyalkoxy)methylgruppe, eine Phenyl-$C_{1-4}$-alkylgruppe oder eine Halophenyl-$C_{1-4}$-alkylgruppe steht, umfassend das zur Reaktionbringen einer Verbindung der Formel

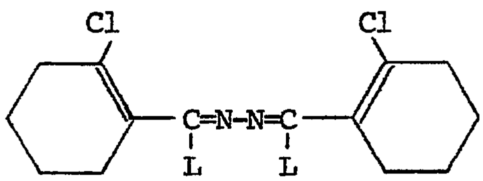

(III)

worin L eine Abgangsgruppe ist, mit einem Hydrazin der Formel $RNH-NH_2$, worin R wie zuvor definiert ist, in Gegenwart eines inerten Lösungsmittels und die Isolierung der gewünschten Verbindung der Formel I, gegebenenfalls, wenn R Wasserstoff ist, gefolgt von einer Reduktion der erhaltenen Verbindung der Formel I, worin A die Gruppe aus Formel II und R Wasserstoff ist, zu der Verbindung von Formel I, worin A eine 1,2,4,5-Tetrazinylengruppe ist.

2. Verfahren nach Anspruch 1, worin R für ein Wasserstoffatom, eine geradkettige $C_{1-4}$-Alkylgruppe, eine Benzylgruppe oder eine 2-Halobenzylgruppe steht, wenn A eine Gruppe der Formel I ist.

3. Verfahren nach Anspruch 1 oder 2, worin R ein Wasserstoffatom oder eine Methyl-, Ethyl-, Benzyl- oder 2-Fluorbenzylgruppe ist, wenn A eine Gruppe der Formel II ist.

4. Verfahren nach Anspruch 1, 2 oder 3, worin L ein Chloratom ist.

5. Verfahren zur Bekämpfung von Schädlingen an einem Ort, umfassend die Applikation einer für die Schädlingsbekämpfung wirksamen Menge einer Verbindung der Formel I nach einem der Ansprüche 1 bis 3.

**Revendications pour les Etats contractants: DE, FR, GB, IT, NL**

1. Une 3,6-disubstitué-1,2,4,5-tétrazine de formule

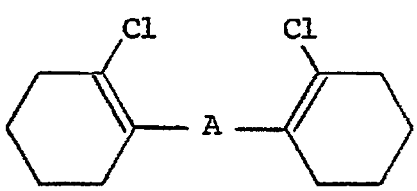

(I)

où A est un groupe 1,2,4,5-tétrazinylène ou est un groupe 1,4-dihydro-1,2,4,5-tétrazinylène de formule

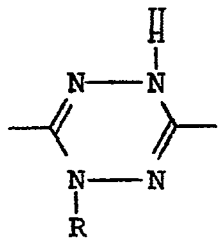

(II)

où R représente un atome d'hydrogène, un groupe alcoyle en $C_{1-4}$, un groupe ($C_{1-6}$ alcoxy)méthyle, un groupe ($C_{2-6}$ alcoxyalcoxy)méthyle, un groupe phényl $C_{1-4}$ alcoyle ou un groupe halophényl $C_{1-4}$ alcoyle.

2. Un composé selon la revendication 1, dans lequel quand A est un groupe de formule II, R représente un atome d'hydrogène, un groupe alcoyle en $C_{1-4}$ à chaîne droite, un groupe benzyle ou un groupe 2-halobenzyle.

3. Un composé selon la revendication 1 ou 2, dans lequel quand Z est un groupe de formule II, R est un atome d'hydrogène ou un groupe méthyle, éthyle, benzyle ou 2-fluorobenzyle.

4. Un procédé de préparation d'un composé de formule I tel que défini dans l'une quelconque des revendications 1 à 3, qui comprend la réaction d'un composé de formule

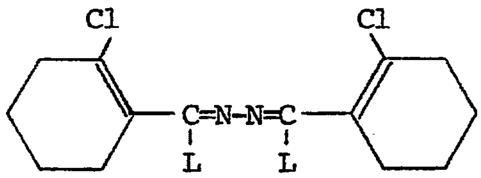

(III)

où L est un groupe qui part, avec une hydrazine de formule $RNH-NH_2$ où R est tel que défini dans la revendication 1, en présence d'un solvant inerte, et l'isolement du composé de formule I désiré, cela étant éventuellement suivi, quand R est de l'hydrogène, d'une réduction du composé de formule I résultant dans lequel A est le groupe de formule II et R est de l'hydrogène pour donner le composé de formule I dans lequel A est un groupe 1,2,4,5-tétrazinylène.

5. Un procédé selon la revendication 4, dans lequel L est un atome de chlore.

6. Une composition pesticide comprenant au moins un véhicule et, comme ingrédient actif, un composé de formule I selon l'une quelconque des revendications 1 à 3.

7. Une composition selon la revendication 6, qui comprend au moins deux véhicules, dont au moins un est un agent tensio-actif.

8. Une méthode de lutte contre des organismes nuisibles en un lieu, qui comprend l'application au lieu concerné d'une quantité efficace du point de vue pesticide d'un composé selon l'une quelconque des revendications 1 à 3 ou d'une composition selon la revendication 6 ou 7.

**Revendications pour l'Etat contractant: ES**

1. Un procédé de préparation d'une 3,6-disubstitué 1,2,4,5-tétrazine de formule

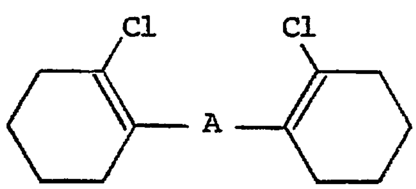

(I)

où A est un groupe 1,2,4,5-tétrazinylène ou est un groupe 1,4-dihydro-1,2,4,5-tétrazinylène de formule

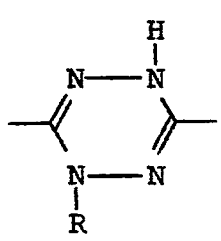

(II)

où R représente un atome d'hydrogène, un groupe alcoyle en $C_{1-4}$, un groupe ($C_{1-6}$ alcoxy)méthyle, un groupe ($C_{2-6}$ alcoxyalcoxy)méthyle, un groupe phényl $C_{1-4}$ alcoyle ou un groupe halophényl $C_{1-4}$ alcoyle, qui comprend la réaction d'un composé de formule

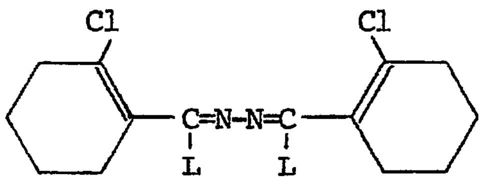

(III)

où L est un groupe qui part, avec une hydrazine de formule $RNH-NH_2$ où R est tel que défini précédemment, en présence d'un solvant inerte, et l'isolement du composé de formule I désiré, cela étant éventuellement suivi, quand R est de l'hydrogène, d'une réduction du composé de formule I résultant dans lequel A est le groupe de formule II et R est de l'hydrogène pour donner le composé de formule I dans lequel A est un groupe 1,2,4,5-tétrazinylène.

2. Un procédé selon la revendication 1, dans lequel quand A est un groupe de formule II, R représente un atome d'hydrogène, un groupe alcoyle en $C_{1-4}$ à chaîne droite, un groupe benzyle ou un groupe 2-halobenzyle.

3. Un procédé selon la revendication 1 ou 2, dans lequel quand A est un groupe de formule II, R est un atome d'hydrogène ou un groupe méthyle, éthyle, benzyle ou 2-fluorobenzyle.

4. Un procédé selon la revendication 1, 2 ou 3, dans lequel L est un atome de chlore.

5. Une méthode de lutte contre des organismes nuisibles en un lieu, qui comprend l'application au lieu concerné d'une quantité efficace du point de vue pesticide d'un composé de formule I tel que défini dans l'une quelconque des revendications 1 à 3.